# EUROPEAN PATENT APPLICATION

(11) **EP 4 552 689 A1**
(43) Date of publication of application: **14.05.2025**
(21) Application number: 24210536.9
(22) Date of filing: 04.11.2024
(51) Int. Cl.: A61N 5/10

(54) **ADMINISTRATION DEVICE**

(30) Priority: 10.11.2023 IT 202300023862
(71) Applicant: Grosso, Maurizio, 12100 Cuneo (CN) (IT); Chauvie, Stephane, 12010 Vignolo (CN) (IT); Cester, Achille, 20090 Opera (MI) (IT); Verificatori Associati Italiani S.r.l., 27055 Rivanazzano Terme (PV) (IT); Cester, Lucrezia, London (GB); Azzaretti Cester, Giovanni Roberto, 27055 Rivanazzano Terme (PV) (IT)
(72) Inventor: CHAUVIE, Stephane, 12010 Vignolo (CN) (IT); CESTER, Lucrezia, London, se207hr (GB); AZZARETTI CESTER, Giovanni Roberto, 27055 Rivanazzano Terme (PV) (IT); GROSSO, Maurizio, 12100 Cuneo (CN) (IT)
(74) Representative: Lunati & Mazzoni S.r.L.

(57) **Abstract**

An administration device (1) configured to be introduced into and housed in an operating cavity (10) located within a patient's body is provided, the device (1) comprising: a flexible tubular element (2) defining an internal cavity (20), a proximal end (21) suitable for placing in fluid passage connection the internal cavity (20) with an environment outside the operating cavity (10) when the device (1) is inserted into the operating cavity (10), and a closed distal end (22) opposite said proximal end (21); the tubular element (2) being suitable for insertion and housed reversibly in the operating cavity (10) and containing a formulation (4) comprising a fluid matrix (40) and active carriers (41) comprising radioactive substances.

## Description

This invention relates to an administration device of the type specified in the preamble of the first claim.

The subject matter of the present invention is an administration device that finds application primarily, but not exclusively, in medical and surgical settings.

In the treatment of cancer diseases, surgical treatment is an elective method, particularly in solid tumors. Exeresis of the tumor mass, however, may not always be complete, as the tumor may have invaded surrounding structures and tissues that cannot be excised.

In addition, the tumor may have infiltrated tissues microscopically. In both cases, treatment of the tumor and its possible dissemination continues by radiotherapy or by chemotherapy/immunotherapy with drug administration.

Drugs are administered according to known methods of administration, such as orally, by injection, or other methods that vary according to the type of drug administered and the treatment prescribed.

In detail, in the case of surgical treatments of tumor tissue removal, relapses may occur.

It is known that the treatment of relapses, or their prevention, can be done by radiotherapy. In fact, through the administration of radioactive substances, any cancer cells that may reform are destroyed, or prevented from forming.

The radioactive substances used typically have characteristics such that they can be as selective as possible with cancer cells.

Radiotherapy methods, depending on the characteristics of the removed tumor, may include different delivery methods.

The most common method involves exposure to ionizing radiation with external beams of the patient's body. This method has the disadvantage of not being able to perform multiple administrations at short time intervals in order to prevent excessive radiation exposure from having adverse effects on the patient. In addition, the waiting time between one administration and the next allows time for tumor formations to regrow.

For these reasons, one administration alternative employed and developed is brachytherapy.

Brachytherapy is a radiation therapy technique in which a radiation source is placed inside the patient's body. The radiation source can be placed in or very close to the tumor to be treated.

Radioactive substances are placed, by means of variously shaped radioactive preparations (needles, wires, seeds), directly into the tumor (interstitial technique), or are placed in anatomical cavities (endocavitary/endoluminal technique). As an alternative, they can be placed on the body surface (contact technique).

Brachytherapy, therefore, involves the administration of a high dose of radiation in a localized manner at or near the tumor site. This reduces the side effects associated with damaging tissues close to the area affected by the tumor, as is the case with traditional radiotherapy, and at the same time the dose of radiation to which the tumor is exposed can be increased, while also reducing the time intervals between administrations.

The ways in which radioactive substances are introduced into the patient's body may be different.

For example, administration can be by injection of a preparation containing microspheres loaded with a radioactive material, or active carriers.

Active carriers containing the radioactive substances can be of the type described in patent IT102022000014707 filed on 13/07/2022, titled "Process for making active carriers."

The prior art just described includes some important drawbacks.

In particular, the known endocavitary or endoluminal administration techniques do not allow the action of the active substances to be limited to the area of interest alone, but there is a risk that the transient areas affected by the route that the active carriers take to reach the site of interest may also be affected by ionizing radiation. This can cause damage to healthy tissues with the risk of further complicating the patient's clinical picture. Moreover, even in this case, the administration must be done in multiple sessions with the consequences already described for external beam radiotherapy.

Finally, the known methods and the devices to implement them do not totally shield medical personnel from exposure to the active substances.

In this situation, the technical task underlying this invention is to devise an administration device capable of substantially overcoming at least part of the aforementioned drawbacks.

Within the scope of said technical task, it is an important purpose of the invention to obtain an administration device that allows therapeutic substances to be administered in a highly localized manner.

Another important purpose of the invention is to make an administration device that can reduce the exposure of healthcare personnel to side effects of administered therapeutic substances.

In addition, another important purpose of the invention is to obtain a user-friendly administration device.

Finally, an important purpose of the invention is to obtain an administration device that does not require multiple treatment sessions.

The technical task and the specified aims are achieved by an administration device as claimed in the annexed claim 1.

Preferred technical solutions are highlighted in dependent claims.

The characteristics and advantages of the invention are clarified below by the detailed description of preferred embodiments of the invention, with reference to the accompanying figures, in which:

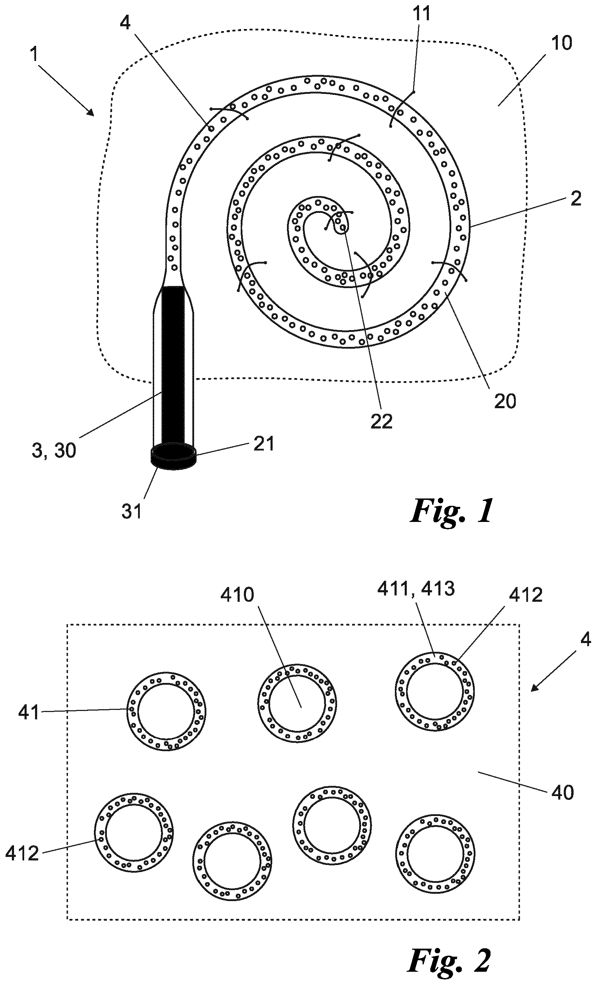

**Fig. 1** shows an initial configuration of an administration device according to the invention;
**Fig. 2** illustrates a detail of one of the configurations of an administration device according to the invention;

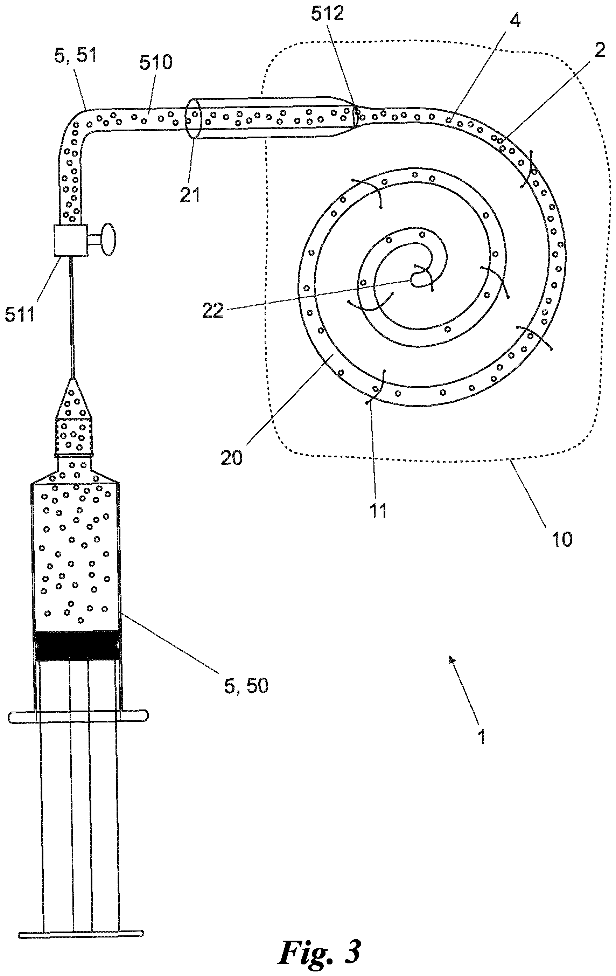

**Fig. 3** illustrates a second configuration of an administration device according to the invention; and

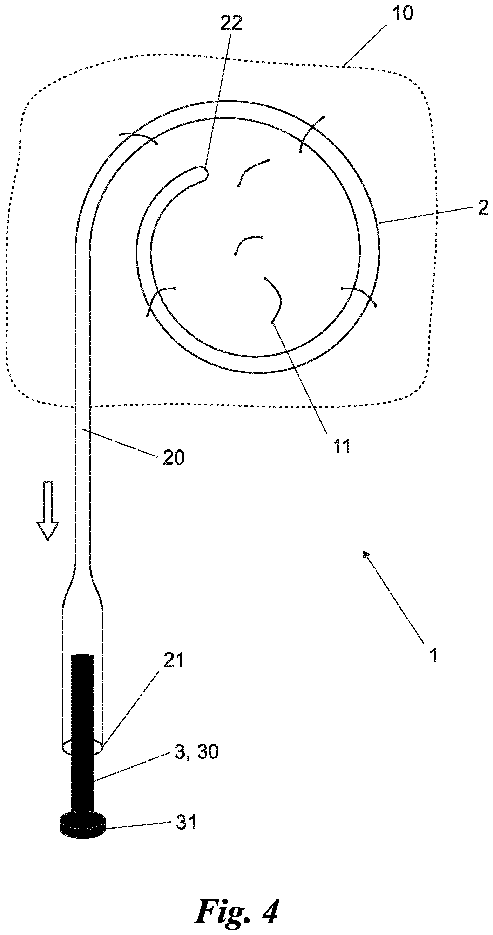

**Fig. 4** shows a third configuration of an administration device according to the invention.

In this document, when measurements, values, shapes, and geometric references (such as perpendicularity and parallelism) are associated with words like "approximately" or other similar terms, such as "almost" or "substantially", they shall be understood as except for errors of measurement or imprecisions due to errors of production and/or manufacturing and, above all, except for a slight departure from the value, measurement, shape, or geometric reference with which it is associated. For example, if associated with a value, such terms preferably indicate a deviation of no more than 10% of the value itself.

Furthermore, when terms such as "first", "second", "upper", "lower", "main", and "secondary" are used, they do not necessarily identify an order, relationship priority, or relative position, but they can simply be used to distinguish different components more clearly from one another.

Unless otherwise specified, as reflected in the following discussions, terms such as "processing", "computing", "determination", "computation", or the like are considered to refer to the action and/or processes of a computer or similar electronic computing device that manipulates and/or transforms data represented as physical, such as electronic quantities of records of a computer system and/or memories, in other data similarly represented as physical quantities within computer systems, records, or other information storage, transmission, or display devices.

The measurements and data reported in this text are to be considered, unless otherwise indicated, as performed in the International Standard Atmosphere ICAO (ISO 2533:1975).

With reference to the figures, the device according to the invention is globally indicated with the number **1.**

Administration device 1 is a device for medical and surgical use. It performs the function of administering substances for pharmaceutical use. Therefore, device 1 performs the function of carrying a drug or substance used in localized therapy. Specifically, device 1 is configured to be introduced into and housed in an operating cavity **10** located within the body of a patient. Operating cavity 10 is an internal area of the body such as, for example, an internal portion of an organ that is the site of surgery. The location within the body, consequent to the identification of the site to be treated by the use of device 1, results from the advantageous use of device 1 in the treatment of diseases that primarily affect organs there. In particular, device 1 is advantageous for use in any tumor exeresis surgery.

In general, operating cavity 10 can be obtained by performing surgery. Therefore, device 1 can be placed inside operating cavity 10 following surgery.

Device 1 includes a flexible tubular element **2.** Tubular element 2 is advantageously configured to be inserted and housed reversibly within operating cavity 10. It performs the function of facilitating the placement of substances for therapeutic use in operating cavity 10. The transport of substances within operating cavity 10 is mediated by the presence of tubular element 2, which not only performs the function of an enclosure containing the therapeutic substance, preventing the substance from spilling out of operating cavity 10, but also performs the function of preventing direct contact of the substance with the walls of operating cavity 10 or the body of a patient.

In particular, tubular element 2 can be inserted inside operating cavity 10, as partly anticipated earlier, following a surgical procedure in which operating cavity 10 and an access to it from outside are obtained. Tubular element 2 is thus the portion of device 1 that is inserted inside operating cavity 10. Notably, surgery can also advantageously be performed solely to place device 1.

Tubular element 2 is preferably constrained to the operating cavity by means of constraint **11.** They may be, for example, stitches. In particular, they serve the function of preventing unwanted displacement of the tubular element 2 within the operating cavity 10. This avoids involving unwanted portions in the administration of therapeutic substances. The means of constraint 11 can be made of biocompatible and biodegradable material so that they can be disposed of within the patient's body without having to be removed later. In addition, the tubular element 2 can remain inside the operating cavity 10 for a limited time depending on the type of therapy to be administered. Therefore, tubular element 2 can be advantageously extracted from operating cavity 10 without the need for a second operation to carry out the removal of tubular element 2. In fact, it can be slipped out of the operating cavity 10 in a similar manner to the removal of drainage catheters.

Specifically, tubular element 2 preferably comprises a polymeric material that is suitable for deformation from a first conformation to a second conformation. The transition from the first conformation to the second conformation is advantageous because it facilitates the production and subsequent deformation operations of the tubular element 2. The transition from the first to the second conformation can be made by deforming the tubular element 2 by means of, for example, a deformable component that can be inserted inside the internal cavity 20 or simply shaped by the operator manually or robotically.

In addition, once inserted inside operating cavity 10, tubular element 2 can be pulled out by pulling.

In detail, the second conformation of tubular element 2 is preferably an at least partially spiral conformation. This form is advantageous since it facilitates removal from operating cavity 10 at the end of treatment by pulling the tubular element 2 from the outside. In fact, as a consequence of the ability of tubular element 2 to deform, spiral conformation can be carried out during the removal operation.

In addition, the spiral conformation makes it possible to optimize the space occupied by tubular element 2. In fact, the folded form of the second spiral conformation allows the concentration of the substance for therapeutic use administered in the occupied area within the operating cavity 10 to be locally increased with the same total volume occupied. In particular, the spiral conformation favors a mainly localized administration on a plane lying on the curve passing through the centers of each cross section of the tubular element 2. This spatial distribution makes administration advantageously more homogeneous.

In detail, the transition from the first to the second conformation occurs in response to a stress. In addition, it maintains the second conformation in the absence of additional external stresses. This feature makes it possible to impart the desired shape to tubular element 2 so that it goes to occupy operating cavity 10 without interfering with portions bordering operating cavity 10 that should not be involved in the action of device 1. Therefore, tubular element 2 performs this function while keeping its shape roughly unchanged in the second conformation. The shape of tubular element 2 does not vary, so it does not experience crushing or deformation as a result of interaction with the walls of operating cavity 10 or with portions near operating cavity 10.

In detail, the polymeric material must then be able to deform plastically. In addition, once deformed under the stress that takes the tubular element 2 from the first to the second conformation, the polymer material retains the new shape. Therefore, the portion of elastic deformation is such that it does not compensate for the overall deformation, resulting in a deformation of the tubular element 2 such that it is permanent, under the regular operating conditions to which it is subjected inside the operating cavity 10, until the operator intervenes from outside to change the conformation subsequently with a new stress.

When extracting tubular element 2 from operating cavity 10, the stress may be such that there is a gradual transition from the second conformation to the first conformation. In detail, this step can be accomplished, for example, when tubular element 2 is pulled out, or pulled, and then pulled out by traction from operating cavity 10, as already anticipated. In this way, tubular element 2 advantageously avoids a second extraction surgery. In addition, traction removal of tubular element 2 turns out to be easier as it requires significantly less invasive surgery for the patient. In fact, it is not required to reopen an access larger than the outer diameter of tubular element 2.

In this regard, the spirally conformed tubular element 2 within the operating cavity 10 has the added advantage of facilitating pull-out as described above.

Specifically, the polymeric material preferably includes polyurethane. This material has the mechanical properties previously described and required for this application. In addition, polyurethane has the advantage of being biocompatible and not interacting with the tissues constituting the walls of the operating cavity 10, avoiding the risk of contamination and infection of the operating cavity 10.

Flexible tubular element 2 defines an internal cavity **20.** It may be a cavity such that it extends along the entire length of the tubular element 2. The internal cavity 20 performs the function of containing the substance for therapeutic use. In addition, it can be used to shape the tubular element from the first to the second conformation before the introduction of the substance. In fact, inside the internal cavity 20 can be inserted the tool that allows the tubular element 2 to be shaped. For example, a curved deformable component can be inserted inside the internal cavity 20.

Tubular element 2 includes a proximal end **21.** It is suitable to place the internal cavity 20 in fluid passage connection with an environment outside the operating cavity 10 when the device 1 is inserted into the operating cavity 10. Therefore, a through-hole is placed at the proximal end 21. In addition, the proximal end 21 can be placed outside the operating cavity 10. In this regard, tubular element 2 is preferably extractable from the operating cavity 10 by pulling the proximal end 21. In fact, the operation of extraction from operating cavity 10 is facilitated if the proximal end 21 protrudes from operating cavity 10. In addition, the deformability of the tubular element 2 causes it to be guided in the extraction operation by the presence of the means of constraint 11. In particular, if the second conformation of the tubular element 2 is of the spiral type, there is a sliding effect of the tubular element driven by the presence of the means of constraint 11 during extraction. The extraction operation, as already anticipated, is thus made advantageously simple. Tubular element 2 includes a distal end **22.** It is preferably closed and opposite the proximal end 21. Therefore, the closed distal end 21 causes the tubular element 2 to be blind and to perform at least a function similar to a container with a single opening in fluid passage connection with the outside such that it can be filled and emptied when required.

Tubular element 2 is advantageously configured to contain a formulation **4.** Formulation 4 includes the therapeutic substance used in the treatment. In particular, formulation 4 comprises a fluid matrix **40** and active carriers **41** comprising radioactive substances.

Fluid matrix 40 performs the function of transporting fluid for the therapeutic substance within flexible element 2, so that there is no local accumulation, but the substance is homogeneously distributed within flexible element 2. For example, fluid matrix 40 may include a saline solution. In this way, it can be used inside a patient's body performing both the function of transportation while reducing the risk of intoxication of the patient.

In detail, the therapeutic substance corresponds to active carriers 41. They preferably include nanospheres **410.** They can be spherical structures made of chemically inert material such as, for example, silica. In fact, nanospheres 410 perform a structural and transport function for radioactive substances. The nanospheres 410 can be surface coated by a layer **411.** It performs the function of keeping radioactive substances adhered to the outer surface of nanospheres 410. In detail, layer 411 comprises an activated powder **412** including radioactive metal oxides. Therefore, activated powder 412 includes radioactive substances, corresponding to these oxides. In addition, layer 411 further includes an organofunctional material **413.** It is designed to disperse and keep chemically bound activated powder 412 and nanospheres 410. Therefore, organofunctional material 413 is also responsible for the transport action carried out by layer 411.

An example of active carriers 41 is described in patent IT102022000014707 filed on 13/07/2022, titled "Process for making active carriers."

In detail, active carriers 41 are nanometer-sized materials optimized for the transport of radionuclide-containing species.

As described above, active carriers 41 typically include nanospheres 410 (Nano, in the present case is a commercially used term that has nothing to do with the order of magnitude so named in the SI, in fact the term microspheres is also used). Nanospheres 410 comprise inorganic material. In detail, nanospheres 410 preferably include materials such as pure silica, to be understood as silica with impurity degree less than 0.1 percent. Nanospheres 410 can also include glass without traces of CO₂ and sodium, or with said elements in amounts of less than 5 percent.

In addition, nanospheres 410 preferably include at least one large internal cavity. Configurations including nanospheres 410 including internal cavities have the advantage of material savings and consequent lightening of active carriers 41.

As anticipated, active carriers 41 comprise an organofunctional material 413 deposited on the surface of nanospheres 410. The organofunctional material 413 preferably includes silanols, which are capable of reacting with the hydroxyl groups (-OR) of the chosen metals, which will be discussed later, with the addition of a functional group, such as the amine group (-NH₂). Alternatively, organofunctional material may include polyethylene. In fact, it too is able to encompass activated powder 412 and ensure the adhesion of layer 411 to the respective nanosphere 410.

Active carriers 41 include an activated powder 412 containing radionuclides. Activated powder 412 is made from a powder 414 that has undergone an activation process, consisting of exposure to neutron beams inside a thermal neutron reactor. Powder 414 preferably includes a metal belonging to the lanthanide series.

In particular, powder 414 preferably includes holmium oxide. This material is known to be effective for applications in the medical field and particularly for therapeutic purposes.

The organofunctional material 413, in preferred solutions, preferably includes silanols that form siloxane bonds.

In technical solutions that include holmium oxide, the average particle diameter falls preferably in a range between 0.05 µm and 10 µm, more preferably between 0.07 µm and 7 µm, even more preferably between 0.1 µm and 5 µm.

The choice of organofunctional material 413 based on the type of powder 414 used is due to the affinity of the powder for the chosen organofunctional material 413.

In some technical solutions, organofunctional material 413 includes at least epoxy-silane and preferably amino-silane. Epoxy-silane is used in this type of technical solution, but the effectiveness of the final active carriers 41 is increased when the coating 5 also includes amino-silane mixed with epoxy-silane by employing a catalyst that speeds up the reaction between the epoxy-silane and the amino-silane to obtain a homogeneous layer.

As anticipated, active carriers 41 comprise a layer 411. Layer 411 includes organofunctional material 413, activated powder 412 and is deposited on the surface of nanospheres 410 according to the steps described in the process.

The function of layer 411 is to enable effective intermediate binding between radionuclides and nanospheres 410, resulting in an enhanced effect on the activity level of radionuclides.

Active carriers 41 are made by a process including a phase of preparation of the nanospheres 410 in inorganic material. At this stage nanospheres are selected that have a diameter that falls preferably in the range of 10 µm to 200 µm, more preferably between 15 µm and 100 µm, more preferably between 20 µm and 60 µm, more preferably between 30 µm and 50 µm, even more preferably between 37 µm and 45 µm. Selection of the nanospheres 410 is preferably done by sieves.

The process includes a step of crushing the coarse matrix from which powder 414 is obtained and grinding it. Crushing of the coarse matrix is preferably done by means of a paddle mill. This stage is advantageous for the purpose of efficiency of active carriers 41. In fact, the grinding process of powder 414 makes it possible to reduce the formation of agglomerates that can become part of the structure in coating 5. The formation of agglomerates in coating 5 causes only part of powder 414 to be converted into activated powder 412, since some of the powder 414 particles are within the agglomerates, so they are shielded from the overlying particles and will expose a smaller area to the neutron beam during the activation phase. This effect has repercussions on the final level of active carrier 41 activity, which will be lower than that attainable when all powder 414 is converted to activated powder 412.

The grinding step involves dispersing the powder 414 in a dispersing agent **8,** preferably including isopropanol or silane gas. The addition of dispersing agent 8 results in improved particle separation of the powder 414, which has the effect of further increasing the final efficiency of active carriers 41. At the end of the grinding stage, a dispersion of the powder 414 is preferably obtained, and the dispersion is poured into a container.

The process includes a step of preparing the organofunctional material 413. At this stage, the chosen organofunctional material 413 is preferably poured into a container.

The process advantageously includes an initial step of synthesizing inactive carriers **7.** Inactive carriers 7 include nanospheres 410, coating 5 deposited on the surface of nanospheres 410, and powder 414 bound to coating 5.

The first stage of synthesis includes a subphase of incorporating the powder 414 into the organofunctional material 413. The incorporation subphase allows the powder 414 to bind to the polymeric material.

The incorporation subphase preferably includes the preparation of a dispersion **6** of the powder 414 in the organofunctional material 413. In variants of the process that include dispersing agent 8 isopropanol in the powder 414 grinding stage, to make dispersion 6, organofunctional material 413 is poured into the mixture containing powder 414 dispersed in dispersing agent 8. In a preferred embodiment to make dispersion 6 dipodal polysiloxane is poured into a mixture of powder 414 in holmium oxide and dispersed in dispersing agent 8 isopropanol.

The first stage of synthesis includes a subphase of deposition of the organofunctional material 413 on the surface of the nanospheres 410 to make the coating 5.

In a preferred variant of the process, dispersion 6, thus including organofunctional material 413, is preferentially deposited on nanospheres 410. In more detail, deposition is preferably done by immersion of the nanospheres 410 in dispersion 6, with subsequent evaporation of the dispersing agent 8. In this way, inactive carriers 7 are obtained, in which powder 414 is bound to nanospheres 410 by coating 5.

In the solution comprising holmium oxide and dipodal polysiloxane, the ratio of the average diameter of holmium oxide particles to the diameter of nanospheres 410 is preferably between 1:10 and 1 :500, more preferably between 1:20 and 1:450, even more preferably between 1:20 and 1:150.

Again with reference to this preferred solution, the percentage ratios between the masses of coating 5 and nanospheres 410 is preferably between 0.05 percent and 6 percent, more preferably between 0.1 percent and 5 percent, even more preferably between 0.1 percent and 4 percent.

In addition, the percentage ratios of holmium oxide masses to nanospheres 410 are preferably between 0.1 percent and 20 percent, more preferably between 0.3 percent and 15 percent, even more preferably between 0.4 percent and 11 percent. The process includes a second step of synthesis of active carriers 41, in which inactive carriers 7 are activated by generation of radionuclides in powder 414, and subsequent transformation of powder 414 into activated powder 412 and consequently of inactive carriers 7 into active carriers 41.

Radionuclide generation is accomplished by exposing the inactive carriers 7 to a neutron beam in order for the neutron radiative capture reaction to take place. The neutron radiative capture reaction leads to the generation of gamma- and/or beta-emitting daughter radionuclides.

Specifically, the activation process is preferably carried out through the use of a thermal neutron reactor by exploiting the radiative neutron capture reaction.

The thermal neutron reactor used in the activation process is preferably the TRIGA (Training Research and Isotopes-production General Atomics) Mark II reactor.

The TRIGA Mark II reactor is a reactor comprising a cooling tank, partly moderated with light water and using fuel composed of 8 percent uranium (enriched to 20 percent), 1 percent hydrogen, and 91 percent zirconium.

The TRIGA Mark II reactor is designed to operate at a maximum steady-state power of 250 kW. The reactor core is shaped like a straight cylinder and contains 90 housings arranged in 5 concentric rings within a circular grid, which also serves the function of a sample holder. The housings contain fuel elements, graphite elements, control rods or radiation channels.

The interior of the reactor includes: a central aluminum cylinder with a diameter of 3.8 cm, placed in the center of the ring containing the fuel elements, a pneumatic "Rabbit" system, placed on the outermost ring that allows the introduction of the sample to be irradiated and its extraction from the reactor, a rotating disk, containing the housings for the cylindrical elements to be inserted inside them, placed inside a circular well with graphite inner walls, which performs the function of a radial reflector, and finally a thermal channel, placed in the tank outside the graphite reflector.

At the beginning of the active carrier 41 synthesis step, inactive carriers 7 are preferably dosed using a tube feeder and weighed inside a polyethylene or quartz container using an analytical scale. The choice of polyethylene or quartz allows easier handling and transport.

The inactive carriers 7 in the polyethylene container, once weighed, are introduced inside another container, suitable for insertion into the thermal neutron reactor. The container containing inactive carriers 7 is placed inside a rotating sample holder housing, preferably positioned to allow irradiation with a neutron flux of 1×10¹² n cm⁻² s⁻¹.

In variants of the process including holmium oxide, irradiation converts at least part of the ¹⁶⁵Ho isotopes contained in holmium oxide to the ¹⁶⁶Ho isotopes. Thus, the neutron radiative capture reaction is ¹⁶⁵Ho (n,y) ¹⁶⁶Ho. The holmium isotope ¹⁶⁵Ho has a relative abundance of 100 percent and a cross section of 64 barns relative to the production of the ¹⁶⁶Ho isotope. The amount of ¹⁶⁶Ho produced depends on the exposure time to neutron irradiation.

Notably, a similar reaction can also occur for sodium typically contained in glass nanospheres 410 as sodium oxide, the reaction ²³Na (n,y) ²⁴Na. The sodium oxide content in glass nanospheres is typically between 12-14%.

If the neutron flux to which the inactive carriers 7 are subjected is higher than 1×10¹² n cm⁻² s⁻¹, the irradiation time must preferably be shortened; in addition, a high neutron flux lends itself to expanding the range of usable radionuclides.

At the end of the radionuclide generation step, powder 414 is converted into activated powder 412 and consequently coating 5 into layer 411. Therefore, inactive carriers 7 are converted to active carriers 41.

Finally, the process includes a subsequent step of analyzing the activity level of active carriers 41.

The analysis step is preferably carried out by gamma spectroscopy. Specifically, in the case of activated powder 412 comprising holmium oxide, the activity level of ¹⁶⁶Ho isotopes of active carriers 41 is measured.

The activity measurement is made after a waiting time after irradiation that is to be measured for the purpose of activity calculation or by measuring an aliquot. This procedure is useful for quality control steps of active carriers 41 once they are produced and analyzed.

The detector used for measurement is preferably a high-purity germanium (HPGe) detector.

The instrumentation for gamma spectroscopy preferably includes a multichannel analyzer, capable of integrating the peak areas of the spectrum. The calibration of energy and efficiency is preferably done by a standard source. The distance between the active carriers and the detector is preferably established based on the expected activity and half-life of each type of radionuclide.

The activity measurement is preferably made by choosing gamma peaks having separate peak areas and net area with an uncertainty of less than 30%.

At the end of the process, active carriers 41 are obtained.

Active carriers 41 according to the invention achieve important benefits.

In fact, this new solution has the advantage of allowing transport of material containing radionuclides in constant quantities. This advantage is due to the better adhesion of the radionuclide-containing powder to the surface of the nanospheres, as well as the spherical shape of the active carriers 41, compared with existing solutions.

Another advantage of this new solution is the easier handling and dosing of active carriers 41 in medical applications. This advantage is due to the free flow property of the glass nanospheres 410.

In addition, this solution has the advantage that no additives are required for the activated powder 412 to adhere to the nanospheres.

Another important advantage is the reduction in the amount of activated powder 412 used, since the improved adhesion of activated powder 412 to the surface of nanospheres 410 allows a higher level of active carriers 41 activity to be achieved for the same amount of powder 414 used at the beginning of the process. This material saving is due to the reduction of powder 414 detachments from the surface of the nanospheres 410.

The savings in material used also implies an economic advantage in choosing this technical solution, since the materials typically chosen for radionuclide generation have high costs.

From the improved adhesion of the activated powder 412 to the surface of the nanospheres 410 also descends the important advantage of reducing dispersion of the powder into the environment and its inappropriate release in the patient. These advantages related to the increased adhesion of the activated powder 412 to the surface of the nanospheres 410 descend from the powder grinding stage 414 for the purpose of avoiding the formation of agglomerates, within which the particles are shielded from exposure to neutron beams during activation.

Another advantage is related to the use of nanospheres comprising silica with impurity degree less than 0.1 percent. In fact, as documented in the table below, the applicant has previously experienced that the use of common glass nanospheres also results in the activation of elements present as impurities in the glass. Activation of impurities results in subsequent undesirable decay processes in the patient and should therefore be avoided. In the table below, the activity values of elements in carrier samples comprising common glass nanospheres are shown:

| **Sample** | **Ho 09** | | **Ho 10** | | **Ho 11** | | **Ho 12** | | **Ho 13** | | **Ho 14** | |
|---|---|---|---|---|---|---|---|---|---|---|---|---|
| **mass (g)** | **0.105** | | **0.1079** | | **0.1057** | | **0.108** | | **0.1098** | | **0.106** | |
| **Isotope** | **MV** | **SD** | **MV** | **SD** | **MV** | **SD** | **MV** | **SD** | **MV** | **SD** | **MV** | **SD** |
| Na-24 | | | | | | | | | 5.85 | 0.19 | 5.40 | 0.19 |
| Sc-46 | 545.14 | 0.03 | 520.27 | 0.03 | 553.78 | 0.03 | 541.97 | 0.03 | 61.52 | 0.04 | 59.15 | 0.05 |
| Cr-51 | 154.15 | 0.06 | 127.92 | 0.07 | 153.24 | 0.07 | 143.01 | 0.07 | 26.83 | 0.25 | 21.19 | 0.28 |
| Fe-59 | 36.64 | 0.09 | 41.48 | 0.09 | 0.00 | 0.00 | 11.32 | 0.26 | 13.42 | 0.17 | 8.14 | 0.27 |
| Co-60 | 9.52 | 0.12 | 4.00 | 0.30 | 10.90 | 0.10 | 9.51 | 0.11 | | | 12.59 | 0.09 |
| Zn-65 | 12.00 | 0.26 | 15.95 | 0.17 | 22.90 | 0.12 | 24.84 | 0.11 | 20.58 | 0.12 | 22.62 | 0.11 |
| Zr-95 | 20.37 | 0.14 | 22.55 | 0.13 | 18.47 | 0.16 | | | | | | |
| Nb-96 | 18.30 | 0.09 | 18.13 | 0.09 | 21.13 | 0.08 | 21.67 | 0.07 | 7.15 | 0.16 | 8.28 | 0.14 |
| Ag-110m | 5.22 | 0.22 | 0.74 | 0.00 | 0.76 | 0.00 | 0.76 | | 19.22 | | 16.93 | |
| Sb-124 | 14.29 | 0.11 | 20.32 | 0.08 | 10.80 | 0.15 | 12.50 | 0.12 | 171.54 | 0.03 | 165.53 | 0.03 |
| Cs-134 | 52.90 | 0.04 | 53.88 | 0.05 | 53.60 | 0.04 | 50.75 | 0.05 | 1.18 | 0.00 | 1.16 | |
| Ba-131 | 9.29 | 0.25 | 14.52 | 0.19 | 15.40 | 0.16 | | | 14.58 | 0.16 | 19.17 | 0.13 |
| Ce-141 | 19.49 | 0.06 | 18.57 | 0.07 | 19.04 | 0.07 | 19.63 | 0.07 | 34.84 | 0.05 | 30.02 | 0.05 |
| Eu-152 | 3.10 | 0.09 | 158.24 | 0.09 | 178.49 | 0.04 | 149.27 | 0.04 | 36.98 | 0.08 | 31.95 | 0.09 |
| Gd-153 | 34.61 | 0.09 | 24.26 | 0.12 | 24.13 | 0.13 | 28.33 | 0.11 | | | | |
| Tb-160 | 25.61 | 0.23 | 20.27 | 0.27 | | | 28.01 | 0.21 | | | | |
| **Ho-166m** | **32.88** | **0.04** | **68.55** | **0.04** | **86.85** | **0.04** | **95.33** | **0.04** | **95.74** | **0.04** | **96.41** | **0.04** |
| Tm-170 | 109.70 | 0.11 | 71.69 | 0.18 | 73.30 | 0.17 | 83.25 | 0.15 | 60.57 | 0.17 | 95.38 | 0.12 |
| Yb-169 | 11.04 | 0.25 | 13.74 | 0.20 | 13.89 | 0.20 | 11.03 | 0.28 | 9.25 | 0.29 | 9.71 | 0.21 |
| Hf-181 | 59.76 | 0.04 | 57.25 | 0.04 | 59.19 | 0.04 | 60.37 | 0.04 | 11.07 | 0.12 | 10.92 | 0.12 |
| Ta-182 | | | 42.67 | 0.12 | 46.10 | 0.11 | 38.82 | 0.13 | 13.45 | 0.27 | | |
| Pa-233 | 42.27 | 0.07 | 41.11 | 0.07 | 41.47 | 0.07 | 44.37 | 0.05 | 7.62 | 0.24 | 7.18 | 0.26 |

| | | | | | | | | | | | | |
|---|---|---|---|---|---|---|---|---|---|---|---|---|
| MV= Mean value (Bq/g) SD= Standard deviation | | | | | | | | | | | | |

As anticipated, active carriers 41 are preferably configured to emit beta radiation.

Beta radiation has the advantage of being less penetrating to tissue than other types of ionizing radiation and, therefore, advantageously reduces the risk of irradiating portions outside the operating cavity 10.

As described above, active carriers 41 preferably include at least one isotope type from a choice of ⁹⁰Y or ¹⁶⁶Ho.

The choice of these two isotopes is therefore preferable since they are effective for this kind of application. In addition, the ⁹⁰Y and ¹⁶⁶Ho isotopes emit beta radiation.

In general, active carriers 41 are advantageous in the treatment of tumors by localized radiotherapy. In particular, active carriers 41 have the advantage of delivering radiation directly to the site of interest. Therefore, by means of device 1, a high concentration of radiation can be delivered directly to the site of interest within the operating cavity 10, reducing the exposure of healthy tissue and thus reducing the side effects of radiation therapy.

In this regard, device 1 is preferably further configured to perform brachytherapy. In detail, device 1 can be used to perform endocavitary or endoluminal brachytherapy if the operating cavity 10 is located within a hollow organ.

In addition, exposure to active carriers 41 can also be carried out at sites that have already undergone cancer tissue resection surgery, so that localized radiotherapy aimed at preventing recurrence can be given.

The proximal end 21 is preferably reversibly attachable to conveying means 5. They are means configured to inject formulation 4 into the internal cavity 20. In addition, the conveying means are configured to extract the formulation 4 from the internal cavity 20.

For example, conveying means 5 may include a syringe **50.**

Conveying means 5 preferably include a coaxial catheter **51.** It is conformed as an additional tubular element with a smaller diameter than that of tubular element 2, so that it can be at least partially inserted within it. Coaxial catheter 51 includes an internal hollow portion **510.** It extends the entire length of the coaxial catheter 51.

Coaxial catheter 51 performs the function of allowing transport of formulation 4 to or from tubular element 2. Therefore, coaxial catheter 51 includes a first end **511** comprising a through-hole and a second end **512,** opposite the first end 511, also comprising a through-hole. The first end 511 can be placed in fluid passage connection with the outlet hole of syringe 50. Alternatively, the exit hole of syringe 50 can be housed inside the first end 511. As another alternative, a needle hooked to the exit hole of syringe 50 can be inserted inside the first end 511. The opening of the first end 511 can be adjusted by a valve.

The second end 512 can be inserted and housed, during the injection and extraction steps of formulation 4, inside cavity 20. Thus, the second end 512 performs the function of allowing passage to or from the internal cavity 20 of formulation 4.

In this regard, the coaxial catheter 51 preferably has a larger diameter than that of the syringe outlet 50. In addition, the diameter of the coaxial catheter 51 is preferably smaller than that of the proximal end 21, so that the coaxial catheter 51 can be inserted within the proximal end 21 and, therefore, the tubular element 2. In this way, coaxial catheter 51 performs the function of conveying formulation 4 from syringe 50 to tubular element 2.

In this regard, the tubular element preferably has an outer diameter between 1 and 5 mm. More preferably, it is between 2 and 4 mm. In addition, tubular element 2 can have variable length depending on the type of operating cavity 10 in which it is housed. For example, it can be between 10 cm and 30 cm in length.

Coaxial catheter 51 can be flexible to facilitate adaptation to the conformation of tubular element 2.

In detail, the coaxial catheter 51 can be inserted within the proximal end 21 and within at least part of the internal cavity 20.

Device 1 preferably includes closure means **3.** They are configured to reversibly plug the proximal end 21. Therefore, they perform the function of confining formulation 4 within cavity 20. They also help prevent any spillage of the formulation 4 from the proximal end 21. The closure means 3 can also be removed, so that the proximal end 21 is again placed in fluid passage connection with the external environment. In this way, formulation 4 can be extracted once the therapeutic treatment is completed.

Closure means 3 may include, for example, an occluder chuck **30.** It can have a cylindrical shape and diameter approximately similar to the inner diameter of the tubular element. In a realization of device 1, the diameter of occluder chuck 30 is larger than the inner diameter of tubular element 2. In this way, a deformation of the tubular element 2 is caused and a tight closure is achieved. By doing so, spillage of formulation 4 from tubular element 2 can be prevented. Occluder chuck 30 may also include a locking end **31.** It is configured to remain outside the tubular element 2. The locking end 31 can have a larger diameter than the remaining part of the occluder chuck 30. The occluder chuck 30, for example, may get stuck in part of the internal cavity 20 due to local plastic deformation of the tubular element 2 and hinder the escape of the formulation 4. The closure end 31 further secures the closure of the proximal end 21 by making contact with the outer edge of the proximal end 21. Occluder chuck 30 can be taken out of the internal cavity 20 when the patient's treatment with formulation 4 is finished.

The operation of device 1 previously described in structural terms is as follows.

Tubular element 2 is initially transitioned from the first conformation to the second conformation. The operation of switching from one conformation to another can be done by inserting a component inside the internal cavity 20. Alternatively, the change of conformation can be made by externally impressed deformation.

The tubular element 2 in the second conformation, which is typically spiral-shaped, is placed inside the operating cavity 10 carved out following an operation in which it is made accessible from the outside through an opening. For example, this operation may be an exeresis of an organ.

Tubular element 2 is inserted empty.

It can be constrained to the walls of the operating cavity 10 by means of sutures, external to the tubular element 2. The portion of the tubular element 2 comprising the proximal end 21 can be positioned so that it partially protrudes from the operating cavity 10. Once tubular element 2 is placed inside operating cavity 10, the open access is closed again to insert tubular element 2 into the patient's body. Next, to insert the formulation 4 inside the tubular element 2, a coaxial catheter 51 can be passed inside the proximal end 21. It is inserted at least partially inside the internal cavity 20. Alternatively, it can be inserted into the proximal end 21 as early as during the introduction of the tubular element 2 inside the operating cavity 10.

In the first end 511 the exit hole of syringe 50 is inserted. Syringe 50 injects formulation 4 inside the coaxial catheter 51 and, passing through the second end 512, passes inside the internal cavity 20. Once formulation 4 is injected, catheter 51 is removed from tubular element 2. It can be removed by slipping it off. To prevent the possible leakage of the formulation 4 from the tubular element 2, the occluder chuck 30 can be inserted inside the tubular element 2. It is embedded and seals the internal cavity 20 containing formulation 4. Active carriers 41 emit radiation that invests the operating cavity 10 for a period determined by the dosage of radiation to be absorbed and the period established for therapy. The residence time of tubular element 2 in operating cavity 10 can last up to one month. At the end of the stipulated period, occluder chuck 30 is removed and formulation 4 can be extracted by surgical drains. Alternatively, another coaxial catheter 51 may be inserted within tubular element 2 and using a syringe 50, similar to that described for injection. In this case, the formulation is passed from the internal cavity 20 to the syringe 50.

Finally, tubular element 2 can be extracted from operating cavity 10 by pulling the portion out of the patient's body. When the end is pulled, the tubular element 2 is gradually unwound in the portion outside the operating cavity 10, while the portion inside the operating cavity 10 is unwound following the spiral conformation imparted, due to the presence of the means of constraint 11 that guide its deformations and the space in which it can move within the cavity.

Device 1 according to the invention achieves important advantages.

In fact, device 1 allows therapeutic substances to be administered in a highly localized manner. Specifically, it allows the introduction of radioactive substances within a restricted volume given by the shape that tubular element 2 can take. The shape can be easily imparted by deforming the tubular element 2. The high concentration of radioactive substances in a localized volume means that device 1 is selective in targeting tissues in the established areas.

Another advantage of device 1 is to avoid the exposure of health care personnel to radioactive substances, since it allows the introduction of formulation 4 and its removal by means of tools that reduce the risks of formulation spillage outside the tubular element 2.

Another advantage of device 1 lies in the ease with which it can be used. In fact, it requires the use of known surgical techniques and allows easier extraction of the tubular element 2 from the operating cavity 10 when the period of exposure to radioactive substances reaches the predetermined term. In fact, it can be removed by slipping it out of the operating cavity 10 with a technique similar to that used for removal of drainage catheters.

In this regard, another important advantage of device 1 is that it does not require a second surgical procedure for removal, since the traction of tubular element 2 is sufficient, which is thus slipped out of the operating cavity 10.

In addition to the administration of radioactive substances, the system can also conveniently be used for in situ administration of chemo and immunotherapeutics in either a single or multiple sessions.

The invention is susceptible of variants falling within the scope of the inventive concept defined by the claims.

In this context, all the details can be replaced by equivalent elements and any materials, shapes and dimensions can be used.

## Claims

1. Device (1) for administration configured to be introduced into and housed in an operating cavity (10) located within the body of a patient,
said device (1) comprising:
- a flexible tubular element (2) defining:
- an internal cavity (20);
- a proximal end (21) suitable for placing in fluid passage connection said internal cavity (20) with an environment external to said operating cavity (10) when said device (1) is inserted into said operating cavity (10); and
- a closed distal end (22) opposite said proximal end (21);
and **characterized by the fact** that:
said tubular element (2) being capable of being inserted and reversibly housed in said operating cavity (10) and containing a formulation (4) comprising a fluid matrix (40) and active carriers (41) comprising radioactive substances.

2. Device (1) according to any one of the preceding claims, wherein said tubular element (2) comprises a material capable of being deformed so as to move from a first conformation to a second conformation in response to a stress and maintain said second conformation in the absence of further external stress.

3. Device (1) according to the preceding claim, wherein said material is a polymeric material.

4. Device (1) according to any one of claims 2-3, wherein said second conformation of said tubular element (2) is an at least partially spiral conformation.

5. Device (1) according to any of the preceding claims, wherein said active carriers (41) comprise nanospheres (410) superficially coated with a layer (411) comprising an activated powder (412) including radioactive metal oxides and an organofunctional material (413) suitable for dispersing and keeping chemically bound said activated powder (412) and said nanospheres (410).

6. Device (1) according to the preceding claim, wherein said active carriers (41) are configured to emit beta radiation.

7. Device (1) according to any of the preceding claims, wherein said active carriers (41) comprise at least one isotope type from a choice of ⁹⁰Y or ¹⁶⁶Ho.

8. Device (1) according to any of the preceding claims, wherein said proximal end (21) is reversibly attachable to conveying means (5) configured to inject said formulation (4) into said internal cavity (20) or to extract said formulation (4) from said internal cavity (20).

9. Device (1) according to any of the preceding claims, comprising closure means (3) configured to reversibly plug said proximal end (21).

10. Device (1) according to any of the preceding claims, wherein said tubular element (2) has an outer diameter having a size between 1 mm and 5 mm.

11. Device (1) according to any of the preceding claims, wherein said tubular element (2) is constrained to said operating lodge (10) by means of constraining elements (11) and said tubular element (2) is removable from said operating lodge (10) by pulling said proximal end (21).
